# EUROPÄISCHE PATENTANMELDUNG

(11) **EP 3 995 821 A1**
(43) Veröffentlichungstag der Anmeldung: **11.05.2022**
(21) Anmeldenummer: 21020559.7
(22) Anmeldetag: 10.11.2021
(51) Int. Cl.: G01N 27/30, G01N 27/333, B01L 3/00, G01N 33/18, G01N 35/08

(54) **MULTI-IONEN-SENSOR, DURCHFLUSS-MESSZELLE UND SYSTEM ZUR MESSUNG VON IONEN IN WÄSSRIGEN SYSTEMEN**

(30) Priorität: 10.11.2020 DE 102020129629
(71) Anmelder: Groß, Matthias, 67663 Kaiserslautern (DE)
(72) Erfinder: Groß, Matthias, 67663 Kaiserslautern (DE)
(74) Vertreter: Völger, Karl Wolfgang

(57) **Zusammenfassung**

Die vorliegende Erfindung bezieht sich auf einen Multi-Ionen-Sensor (1) zur Messung von Ionen in wässrigen Systemen, umfassend
- einen flexiblen oder semi-flexiblen Träger (11),
- eine auf den Träger (11) aufgebrachte Referenzelektrode (13),
- mindestens drei auf den Träger (11) aufgebrachte Arbeitselektroden (15a, 15b, 15c),
wobei der Träger (11), die Referenzelektrode (13) und die Arbeitselektroden (15a, 15b, 15c) eine ionenselektive Sensoreinheit (101) bilden,
- eine Anschlusseinrichtung (17) für die ionenselektive Sensoreinheit (101) und
- ein Sensorgehäuse (19), welches eine Baugruppe aus ionenselektiver Sensoreinheit (101) und Anschlusseinrichtung (17) aufnimmt,
wobei die Referenzelektrode (13) Abmessungen von max. 4 mm x 4 mm x 4 mm aufweist.

Die vorliegende Erfindung bezieht sich ferner auf eine Durchfluss-Messzelle (3) für die Messung von Ionen in wässrigen Systemen sowie auf ein entsprechendes Verfahren und auf ein System zur Messung von Ionen in wässrigen Systemen und zum Einstellen der lonengehalte in den wässrigen Systemen.

## Beschreibung

Die vorliegende Erfindung bezieht sich auf einen Multi-Ionen-Sensor, eine Durchfluss-Messzelle und ein System zur Messung von Ionen in wässrigen Systemen sowie auf ein entsprechendes Verfahren.

Gattungsgemäße Multi-Ionen-Sensoren und entsprechende Systeme zur Messung von Ionen in wässrigen Systemen sind aus dem Stand der Technik grundsätzlich bekannt. Allen diesen bekannten Geräten ist gemeinsam, dass sie Einzelelektroden in sog. "Solid State"-Technik, insbesondere in Form von Carbon-Röhrchen, für jedes zu bestimmende Ion und für die Referenz aufweisen, so dass der eigentliche Multi-Ionen-Sensor durch die Mehrzahl an Einzelelektroden sehr groß und kostspielig ist. Ferner ist bei dieser Art von Multi-Ionen-Sensoren eine Zwischenreinigung der Elektroden bei der Messung nötig, z.B. mit destilliertem Wasser. Schließlich werden sowohl Probe als auch Referenzflüssigkeiten meist in einen offenen Behälter gepumpt, in dem der gattungsgemäße Multi-Ionen-Sensoren eingesetzt ist, weshalb das zu messende Flüssigkeitsvolumen relativ groß ist. Schließlich muss die Flüssigkeit nach den Kalibrierungen und nach der Messung und aus dem Behälter wieder abgepumpt werden und erzeugt ein nicht geringes Abfallvolumen.

Es besteht daher Bedarf an einem neuartigen Multi-Ionen-Sensor und einem entsprechenden Mess-System, mit dem die Nachteile des Standes der Technik überwunden werden können.

Der vorliegenden Erfindung liegt daher die Aufgabe zugrunde, einen Multi-Ionen-Sensor, eine Durchfluss-Messzelle und ein System zur Messung von Ionen in wässrigen Systemen bereitzustellen und ein entsprechendes Verfahren anzugeben, welche kostengünstiger als die bekannten Systeme sind und gleichzeitig weniger Messvolumen benötigen. Ein weiteres Ziel ist eine präzisere Messung.

Diese Aufgabe wird in einem ersten Aspekt der vorliegenden Erfindung durch einen Multi-Ionen-Sensor (1) zur Messung von Ionen in wässrigen Systemen gelöst, der umfasst
- einen flexiblen oder semi-flexiblen Träger (11),
- eine auf den Träger (11) aufgebrachte Referenzelektrode (13),
- mindestens drei auf den Träger (11) aufgebrachte Arbeitselektroden (15a, 15b, 15c),
wobei der Träger (11), die Referenzelektrode (13) und die Arbeitselektroden (15a, 15b, 15c) eine ionenselektive Sensoreinheit (101) bilden,
- eine Anschlusseinrichtung (17) für die ionenselektive Sensoreinheit (101) und
- ein Sensorgehäuse (19), welches eine Baugruppe aus ionenselektiver Sensoreinheit (101) und Anschlusseinrichtung (17) aufnimmt,
wobei die Referenzelektrode (13) Abmessungen von max. 4 mm x 4 mm x 4 mm aufweist.

Unter "Multi-Ionen-Sensor" wird erfindungsgemäß verstanden, dass der Sensor dazu ausgelegt ist, verschiedene Ionen (d.h. Kationen und Anionen) in einem wässrigen System zu messen.

Der Träger (11) ist erfindungsgemäß flexibel oder semi-flexibel ausgestaltet, was durch die Verwendung beispielsweise einer dünnen flexiblen Leiterplatte sichergestellt wird.

Die Referenzelektrode (13) weist ein konstantes, sich schnell und reproduzierbar einstellendes Gleichgewichtspotential auf, das als Bezugspunkt für die Messung der relativen Potentiale der Arbeitselektroden (15a, 15b, 15c) dient.

Die erfindungsgemäß eingesetzten Arbeitselektroden (15a, 15b, 15c) sind auf spezielle Ionen (Kationen und Anionen) ausgerichtet, wobei an deren Oberflächen elektrochemische Vorgänge in kontrollierter Weise ablaufen, deren Potential im Vergleich zur Referenzelektrode (13) bestimmt wird.

Die erfindungsgemäß als ionenselektive Sensoreinheit (101) bezeichnete Einheit umfasst die Referenzelektrode (13) und die Arbeitselektroden (15a, 15b, 15c), die auf den Träger (11) aufgebracht sind.

Die Anschlusseinrichtung (17) dient erfindungsgemäß dazu, die Sensoreinheit (101) aufzunehmen und elektrisch zu kontaktieren. Hierzu kann die Sensoreinheit (101) eine Kontaktfläche am gegenüberliegenden Ende der Elektrodenenden aufweisen, die in eine entsprechende Aufnahme der Anschlusseinrichtung (17) eingeschoben wird.

Zweckmäßigerweise umfasst die Anschlusseinrichtung (17) ferner ein Datenkabel, das am Ende mit einem Stecker versehen ist.

Das Sensorgehäuse (19) dient im Wesentlichen dem Schutz der Sensoreinheit (101) vor Feuchtigkeit und mechanischen Einflüssen. Seine Außenmaße sind zudem auf die Durchfluss-Messzelle (3) abgestimmt.

Erfindungswesentlich ist die Referenzelektrode (13), die eine gegenüber dem Stand der Technik minimale Baugröße von max. 4 mm x 4 mm x 4 mm aufweist. Die Untergrenze der Baugröße der Referenzelektrode (13), liegt bei der technisch derzeit realisierbaren Größe und kann mit 0,5 mm x 0,5 mm x 0,5 mm angegeben werden. Die besonders bevorzugte Größe liegt zwischen 1,8 mm x 1,8 mm x 1,8 mm und 2,2 mm x 2,2 mm x 2,2 mm.

Zudem besitzt die erfindungsgemäße Referenzelektrode (13) eine dem Stand der Technik im Hinblick auf Pseudo-Referenzelektroden überlegene Präzision, während sie hinsichtlich echter Referenzelektroden nach dem Stand der Technik weniger Aufwand, reduzierte Größe und geringere Kosten benötigt.

Schließlich handelt es sich bei der erfindungsgemäßen Referenzelektrode (13) um eine echte Referenzelektrode und keine Pseudo-Referenzelektrode, wie im Stand der Technik meist verwendet. Mit der echten Referenzelektrode (13) der vorliegenden Erfindung wird ein konstantes Potential erzeugt, das unabhängig von der Probenlösung ist. Bei Pseudo-Referenzelektroden handelt es sich dagegen normalerweise um metallische Leiter (z.B. als Metalldrähte), die direkt in eine Elektrolytlösung / Probenlösung getaucht werden. Zwar stellt sich an solchen Pseudo-Referenzelektroden auch ein konstantes Potential ein, dies ist allerdings unbekannt und von der Zusammensetzung der Elektrolytlösung / Probenlösung abhängig.

Die vorliegende Erfindung hat im Rahmen des Multi-Ionen-Sensors (1) die Vorteile, einer deutlich kleineren Bauweise verglichen mit den bekannten Sensoren, was durch das Aufdrucken der Arbeitselektroden und der Applikation der echten Miniatur-Referenzelektrode (13) ermöglichst wird. Zudem ist der erfindungsgemäße Multi-Ionen-Sensor (1) deutlichen kostengünstiger als ein Sensor bestehend aus den bekannten Solid-State-Einzelelektroden und einer separaten Referenzelektrode.

Erfindungswesentlich ist weiter, dass die echte Referenzelektrode (13) (keine Pseudo-Referenzelektrode!) und die Arbeitselektroden (15a, 15b, 15c) in der miniaturisierten Form integral auf einem Bauteil angeordnet sind, in der vorliegenden Erfindung wird dieses integrierte Bauteil als ionenselektive Sensoreinheit (101) bezeichnet.

In einer Weiterbildung des vorliegenden Multi-Ionen-Sensors (1) ist das Material der Referenzelektrode (13) ausgewählt aus polymerbeschichteten Fasern und Ag/AgCl.

Eine Ausführungsform des erfindungsgemäßen Multi-Ionen-Sensors (1) sieht vor, dass das Sensorgehäuse (19) die Baugruppe aus ionenselektiver Sensoreinheit (101) und Anschlusseinrichtung (17) so aufnimmt, dass die Rückseite des Trägers (11) auf einem Stützteil (191) des Sensorgehäuses (19) aufliegt, und dass das Sensorgehäuse (19) die Baugruppe aus ionenselektiver Sensoreinheit (101) und Anschlusseinrichtung (17) soweit umschließt, dass die Enden der Referenzelektrode (13) und der Arbeitselektroden (15a, 15b, 15c) gegenüber der Außenseite freiliegen und damit den Messkopf (103) des Multi-Ionen-Sensors (1) bilden.

Mit dieser Ausgestaltung wird zunächst sichergestellt, dass der mechanisch empfindliche Träger (11) ausreichend gestützt wird, um für die Messung zur Verfügung zu stehen.

Die vorstehend dargestellte Ausführungsform kann dergestalt weitergebildet werden, dass das Sensorgehäuse (19) eine rückspringende Teilfläche (193) aufweist, welche geometrisch mit dem Stützteil (191) mit darauf aufliegender ionenselektiver Sensoreinheit (101) korrespondiert, so dass zwischen der ionenselektiven Sensoreinheit (101) auf dem Stützteil (191) und der rückspringenden Teilfläche (193) ein Messspalt (S) gebildet wird.

Diese Ausgestaltung mit der Schaffung eines Messspalts (S) verbessert den Fluss der zu messenden Flüssigkeit über die ionenselektive Sensoreinheit (101).

Eine spezielle Ausführungsform sieht ein zweiteiliges Sensorgehäuse (19) vor,
wobei eine erste Sensorgehäuse-Hälfte die Baugruppe aus ionenselektiver Sensoreinheit (101) und Anschlusseinrichtung (17) so aufnimmt, dass die Rückseite des Trägers (11) auf einem Stützteil (191) der ersten Sensorgehäuse-Hälfte aufliegt,
wobei eine zweite Sensorgehäuse-Hälfte die Baugruppe aus ionenselektiver Sensoreinheit (101) und Anschlusseinrichtung (17) soweit umschließt, dass die Enden der Referenzelektrode (13) und der Arbeitselektroden (15a, 15b, 15c) gegenüber der Außenseite freiliegen und damit den Messkopf (103) des Multi-Ionen-Sensors (1) bilden.

Es hat sich insbesondere als vorteilhaft herausgestellt, wenn die Höhe (H) des Messspalts (S) und die Breite (B) des Messspalts (S) in einem geometrischen Verhältnis H : B von 1 : 1 bis 4 : 1 stehen, da auf diese Weise eine laminare Strömung in dem Messspalt (S) erzeugt wird.

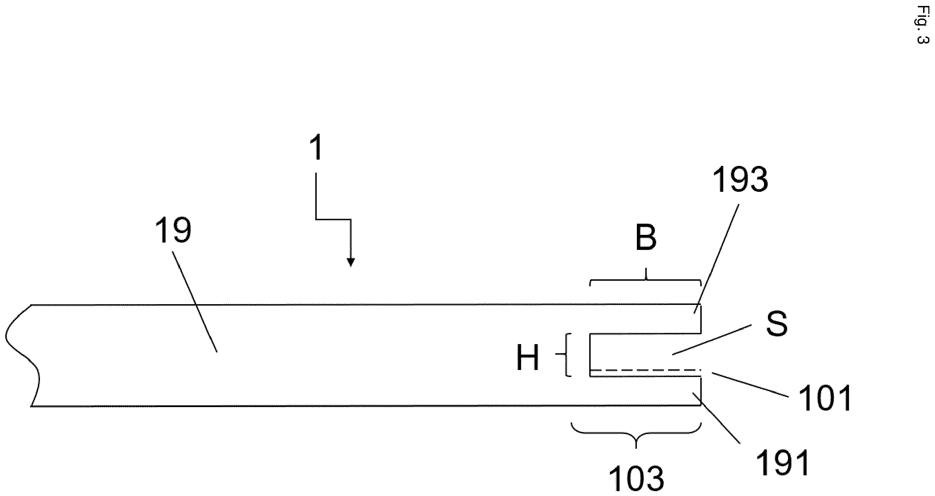

Die Höhe (H) des Messspalts (S) ergibt sich aus dem Abstand zwischen der auf dem Stützteil (191) aufliegenden ionenselektiven Sensoreinheit (101) und der rückspringenden Teilfläche (193). Die Breite (B) des Messspalts (S) entspricht dem offenen Maß in Längsrichtung des Multi-Ionen-Sensors (1), die in Figur 3 dargestellt.

Die vorstehend genannte Aufgabe wird in einem zweiten Aspekt der vorliegenden Erfindung durch eine Durchfluss-Messzelle (3) für die Messung von Ionen in wässrigen Systemen gelöst, welche umfasst
- eine Aufnahmeöffnung (31) zur Montage eines erfindungsgemäßen Multi-Ionen-Sensors (1), wie er vorstehend beschrieben wurde,
wobei der Messkopf (103) des Multi-Ionen-Sensors (1) formschlüssig in der Aufnahmeöffnung (31) montierbar ist und zusammen mit der Rückwand der Aufnahmeöffnung (31) einen Durchfluss-Messraum bilden kann,
- zumindest eine Kalibrier-Zuleitung (33),
- zumindest eine Proben-Zuleitung (35),
- eine Sammelleitung (37), welche die zumindest eine Kalibrier-Zuleitung (33) und die zumindest eine Proben-Zuleitung (35) aufnimmt und sich zu der Aufnahmeöffnung (31) hin erstreckt,
- eine Ableitung (39), die sich oberhalb an die Aufnahmeöffnung (31) anschließt.

Bei der Aufnahmeöffnung (31) handelt es sich insbesondere um eine Ausfräsung, deren Kontur der Außenkontur des Multi-Ionen-Sensors (1) entspricht, so dass dieser formschlüssig ausgenommen werden kann. Die Enden des Messkopfs (103) liegen dabei an der Rückwand der Aufnahmeöffnung (31) an, so dass der Durchfluss-Messraum gebildet wird, der hauptsächlich aus dem Messspalt (S) und den direkt hineinführenden Zuleitungen besteht.

Zur weiteren Sicherung, insbesondere gegen ein Verdrehen, kann der Messkopf (103) des Multi-Ionen-Sensors (1) seitliche Nuten aufweisen, in welche passende Führungsschienen eingreifen, die auf der Innenseite der Aufnahmeöffnung (31) vorgesehen sind. Das Sichern gegen Verdrehen ist besonders dafür wichtig, dass der Messspalt (S) zum Ausbilden einer laminaren Strömung exakt justiert wird.

Bei der Kalibrier-Zuleitung (33) und der Proben-Zuleitung (35) handelt es sich insbesondere um Bohrungen / Fräsungen, an deren äußerem Ende entsprechende Anschlüsse (z.B. Schlauchmuffen) vorgesehen sind. An ihrem inneren Ende führen die Zuleitungen (33, 35) in die Sammelleitung (37), welche zu der Aufnahmeöffnung (31) hin ausgerichtet ist.

Insbesondere mündet die Sammelleitung (37) in den unteren Bereich der Aufnahmeöffnung (31), so dass die durchgeführte Flüssigkeit von unten in den Durchfluss-Messraum, bzw. in den Messspalt (S) eingeleitet wird und eine laminare Strömung ausbilden kann.

Bei der Ableitung (39) handelt es sich ebenfalls insbesondere um eine Bohrung / Fräsung, die vorteilhafterweise oberhalb des Durchfluss-Messraums, bzw. des Messspalts (S) angeordnet ist.

Als besonderes Merkmal kann am Übergang von der Aufnahmeöffnung (31) in die Ableitung (39) ein erweiterter Hohlraum vorgesehen sein, um etwa auftretende Luftblasen zu sammeln und damit die präzise Messung nicht zu verfälschen.

Die erfindungsgemäße Durchfluss-Messzelle (3) weist zunächst den Vorteil auf, dass sie keinen offenen Behälter darstellt, der gefüllt und wieder geleert werden muss. Einflüsse der Umgebungsluft sowie Verdampfung und damit einhergehende Konzentrationsschwankungen werden so unterbunden. Ferner weist die erfindungsgemäße Durchfluss-Messzelle (3) ein minimales Volumen und damit sehr geringe Strömungs-Toträume auf, eine Selbstentlüftung kann vorgesehen sein. Diese Merkmale führen zu der Möglichkeit, mit hohes Messgenauigkeit bei kleinem Probenvolumen und einfacher 2-Punkt Kalibrierung die Messungen durchzuführen. Schließlich sind erfindungsgemäße keine Zwischenspülungen notwendig.

Eine Weiterbildung der erfindungsgemäßen Durchfluss-Messzelle (3) sieht vor, dass die Durchmesser der Kalibrier-Zuleitung (33) und/oder der Proben-Zuleitung (35) und/oder der Sammelleitung (37) 2 mm bis 4 mm betragen.

Hierdurch wird für die Durchfluss-Messzelle (3) ein gegenüber dem Stand der Technik, deutlich reduziertes Volumen sowohl für die Probe als auch für die Kalibrierflüssigkeiten erreicht. Dies trägt einerseits zum sparsamen Umgang mit den eingesetzten Flüssigkeiten für die Referenzen bei, andererseits ist der erfindungsgemäße Multi-Ionen-Sensor (1) darauf ausgelegt, auch bei sehr geringen Volumina äußerst präzise zu messen.

Insbesondere beträgt das kumulierte Volumen der Kalibrier-Zuleitung (33), der Proben-Zuleitung (35), der Sammelleitung (37) und des Durchfluss-Messraums 2 ml bis 5 ml.

Ein dritter Aspekt der vorliegenden Erfindung zur Lösung der vorstehend genannten Aufgabe bezieht sich auf ein Verfahren zur Messung von Ionen in wässrigen Systemen, umfassend die Schritte
a) Bereitstellen einer erfindungsgemäßen Durchfluss-Messzelle (3), wie sie vorstehend beschrieben wurde, mit einem montierten erfindungsgemäßen Multi-Ionen-Sensor (1), wie sie vorstehend beschrieben wurde,
b) Einleiten einer ersten wässrigen Kalibrierlösung in die Durchfluss-Messzelle (3) mit Umspülen der ionenselektiven Sensoreinheit (101) des Multi-Ionen-Sensors (1), dabei Ausführen einer ersten Kalibrierung und Aufnehmen eines ersten Referenz-Messwerts,
c) Einleiten einer wässrigen Probe in die Durchfluss-Messzelle (3) mit Umspülen der ionenselektiven Sensoreinheit (101) des Multi-Ionen-Sensors (1), dabei Aufnehmen eines Messwertebündels,
d) Einleiten einer zweiten wässrigen Kalibrierlösung in die Durchfluss-Messzelle (3) mit Umspülen der ionenselektiven Sensoreinheit (101) des Multi-Ionen-Sensors (1), dabei Ausführen einer zweiten Kalibrierung und Aufnehmen eines zweiten Referenz-Messwerts,
wobei die Schritte a), b) c) und d) quasi kontinuierlich ausgeführt werden, ohne die Durchfluss-Messzelle (3) zu entleeren,
e) Berechnen der Messwerte aus ersten und zweiten Referenz-Messwerten und Messwertebündel,
f) Verarbeitung der Messwerte in einer Recheneinheit.

In den Schritten b), c) und d) werden die jeweiligen Flüssigkeiten nicht aktiv abgeleitet, sondern es ergibt sich automatisch durch das Einleiten der nächsten Flüssigkeit eine Verdrängung. Aufgrund der geringen Querschnitte der Leitungen und der damit verbundenen geringen Volumina ist eine solches Vorgehen möglich, ohne dass sich die aufeinander folgenden Flüssigkeiten in nennenswertem Umfang vermischen. Auch aus diesem Grund kann auf ein Zwischenspülen verzichtet werden.

Bei der ersten wässrigen Kalibrierlösung kann es sich insbesondere um Wasser mit lonenkonzentrationen, die etwas unter den erwarteten Konzentrationen liegen, handeln.

Die wässrige Probe ist in einer besonders bevorzugten Ausführungsform das Wasser eines Aquariums.

Bei der zweiten wässrigen Kalibrierlösung kann es sich insbesondere um Wasser mit lonenkonzentrationen, die etwas über den erwarteten Konzentrationen liegen, handeln.

Für die Ausführung der Erfindung hat es sich als besonders vorteilhaft herausgestellt, wenn die Konzentration der verschiedenen Ionen in der Probe zwischen den jeweiligen Konzentrationen der ersten und zweiten wässrigen Kalibrierlösung liegen.

Bei der Messung in Schritt c) wird von jeder der einzelnen Arbeitselektroden (15a, 15b, 15c) ein für ein Ion (Anion oder Kation) spezifischer Messwert erfasst, die Gesamtheit dieser spezifischen Messwerte ergibt das Messwertbündel.

Die vorliegende Erfindung hat im Rahmen des Verfahrens grundsätzlich die vorstehend bereits beschriebenen Vorteile, dass mit dem erfindungsgemäßen Multi-Ionen-Sensor (1) und der erfindungsgemäßen Durchfluss-Messzelle (3) eine deutlich kostengünstigere Messung, verglichen mit dem Stand der Technik, ermöglicht wird. Die Messung wird durch die kleinen Volumina präziser und ist leichter und einfacher durchzuführen.

Es hat sich in einer Weiterbildung des erfindungsgemäßen Verfahrens als vorteilhaft herausgestellt, wenn für das erfindungsgemäße Verfahren das zur Messung benötigte Volumen der ersten wässrigen Kalibrierlösung, der wässrigen Probe und der zweiten wässrigen Kalibrierlösung 3 ml bis 10 ml beträgt.

Mit einem solchen, recht geringen Volumen wird aufgrund der erfindungsgemäß geringen Abmessungen von Multi-Ionen-Sensor (1) und Durchfluss-Messzelle (3) trotzdem eine präzise Messung ermöglicht.

Es hat sich für eine noch präzisere Messung als vorteilhaft herausgestellt, wenn vor Schritt b) das in den Zuleitungen zur Durchfluss-Messzelle (3) vorhandene Flüssigkeitsvolumen vollständig durch frisches Flüssigkeitsvolumen des wässrigen Systems ausgetauscht wird, wobei das ausgetauschte vorhandene Flüssigkeitsvolumen dem wässrigen System wieder zugeführt wird.

Einerseits wird hierdurch frische zu messende Flüssigkeit bereitgestellt, andererseits wird diese Flüssigkeit nicht verworfen und damit verschwendet, was einerseits kostenschonend und andererseits umweltfreundlich ist.

Schließlich bezieht sich die vorliegende Erfindung in einem vierten Aspekt zur Lösung der vorstehend genannten Aufgabe auf ein System zur Messung von Ionen in wässrigen Systemen und zum Einstellen der Ionengehalte in den wässrigen Systemen, umfassend
- eine erfindungsgemäßen Durchfluss-Messzelle (3), wie sie vorstehend beschrieben wurde,
- einen in der Durchfluss-Messzelle (3) montierten erfindungsgemäßen Multi-Ionen-Sensor (1), wie er vorstehend beschrieben wurde,
- eine Pumpeneinheit (5), welche eingangsseitig zur Entnahme der Probe mit dem wässrigen System und mit Vorratsbehältern für die erste und zweite wässrige Kalibrierlösung fluiddynamisch verbunden ist und welche ausgangsseitig mit der zumindest einen Kalibrier-Zuleitung (33) und der zumindest einen Proben-Zuleitung (35) fluiddynamisch verbunden ist,
- eine Steuer-/Regel-Einheit (7), welche eingangsseitig mit dem Multi-Ionen-Sensor (1) elektronisch verbunden ist,
wobei die Steuer-/Regel-Einheit (7) eine Auswerte-Elektronik zur Verarbeitung der ersten und zweiten Referenz-Messwerte und Messwertebündel aufweist.

Die Pumpeneinheit (5) ist mehrkanalig aufgebaut und weist erfindungsgemäß separat ansteuerbare Dosierpumpen für kleine Volumina auf. Diese Dosierpumpen sind unabhängig voneinander steuerbar.

Bei der Steuer-/Regel-Einheit handelt es sich erfindungsgemäß um beispielsweise einen Aquarien-Computer, der Informationen der Messungen von Ionen-Konzentrationen verarbeitet und in Abhängigkeit von der Regeldifferenz (Soll zu Ist) der Ionen-Konzentration über geeignete Maßnahmen (z.B. Änderung der Ionen-Zufuhr durch Steuerung von Dosierpumpen oder Magnetventilen) die Ionen-Konzentration reguliert.

Das erfindungsgemäße System weist grundsätzlich die vorstehend bereits beschriebenen Vorteile auf, nämlich dass mit dem erfindungsgemäßen Multi-Ionen-Sensor (1) und der erfindungsgemäßen Durchfluss-Messzelle (3) eine deutlich kostengünstigere Messung, verglichen mit dem Stand der Technik, ermöglicht wird. Die Messung wird durch die kleinen Volumina präziser und ist leichter und einfacher durchzuführen.

Eine Weiterbildung des Systems sieht vor, dass die Steuer-/Regel-Einheit dazu ausgelegt ist, die Ionen-Konzentration in dem wässrigen System in Abhängigkeit der Regeldifferenz zu regulieren.

Das erfindungsgemäße System ermöglicht hierdurch eine vollautomatische Regelung, bei welcher eine einstellbare Regeldifferenz (Abweichung Ist-Wert zu Soll-Wert der einzelnen Ionen) eine einstellbare Nachjustierung von Dosiermengen von Dosierpumpen bewirken kann.

Weitere Ziele, Merkmale, Vorteile und Anwendungsmöglichkeiten ergeben sich aus der nachfolgenden Beschreibung von die Erfindung nicht einschränkenden Ausführungsbeispielen anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigen:
- Fig. 1: eine schematische Darstellung der erfindungsgemäßen ionenselektiven Sensoreinheit 101 gemäß einer Ausführungsform der Erfindung,
- Fig. 2: eine schematische Darstellung des erfindungsgemäßen Multi-Ionen-Sensor 1 gemäß einer Ausführungsform der Erfindung in offenem Zustand,
- Fig. 3: eine schematische Darstellung des erfindungsgemäßen Multi-Ionen-Sensor 1 gemäß einer Ausführungsform der Erfindung in geschlossenem Zustand und
- Fig. 4: eine schematische Schnittdarstellung der erfindungsgemäßen Durchfluss-Messzelle 3 gemäß einer Ausführungsform der Erfindung.

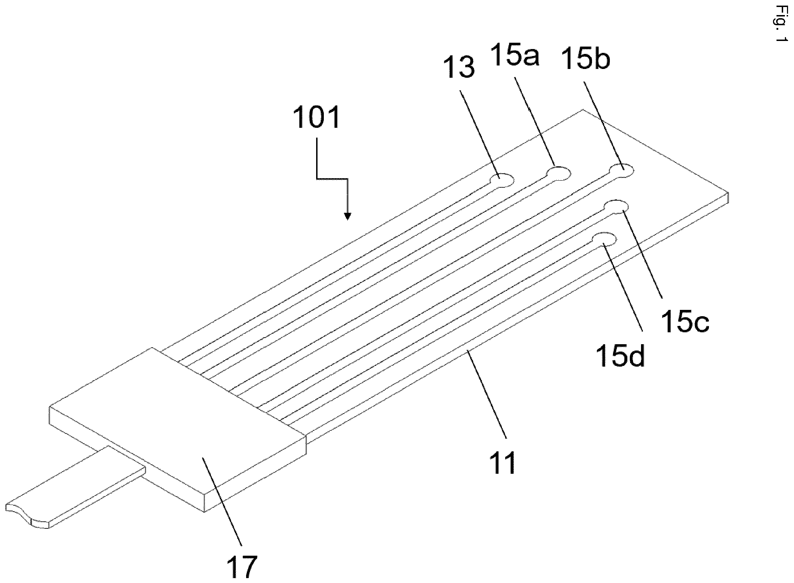

In Figur 1 wird schematisch die ionenselektive Sensoreinheit 101 in einer bevorzugten Ausführungsform der Erfindung dargestellt. Auf den Träger 11, hier eine flexible Leiterplatte, sind mechanisch die Elektroden aufgedruckt, beispielsweise als Graphitbahnen. In der vorliegenden Ausführungsform sind vier der gedruckten Elektroden als Arbeitselektroden 15a, 15b, 15c, 15d ausgeführt, wobei der Großteil der Leiterbahnen zum Schutz mit einem Lack abgedeckt ist und im Wesentlichen das in der Figur rund dargestellte Ende den Messpunkt bildet. Abhängig von den zu messenden Ionen sind die Messpunkte entsprechend mit einem ionensensitiven Material ausgebildet.

Die in dieser Darstellung fünfte Elektrode stellt die echte Referenzelektrode 13 dar, die bezüglich Ihrer Zuleitung ebenfalls zunächst aus einer aufgedruckten Leiterbahn (z.B. Graphit) besteht. Am rund dargestellten Messpunkt wird jedoch die eigentliche, miniaturisierte Elektrode aufgebracht, bzw. kontaktiert, wobei das Material besonders bevorzugt aus polymerbeschichteten Fasern besteht, welche mit Ag/AgCl (Silber/Silberchlorid) versetzt sind.

Die Anordnung bzw. Abfolge der Referenzelektrode 13 und der Arbeitselektroden 15a, 15b, 15c, 15d in Figur 1 ist lediglich beispielhaft und legt die Erfindung nicht auf die konkrete Darstellung fest.

Die ionenselektive Sensoreinheit 101 wird an dem den Messpunkten gegenüber liegenden Ende des Trägers 11 durch eine Anschlusseinrichtung 17 kontaktiert. In bevorzugter Ausführungsform weisen die aufgedruckten Leiterbahnen am Ende eine Kontaktfläche auf, mit welcher sie in die Anschlusseinrichtung 17 eingesteckt werden können. Die Anschlusseinrichtung 17 weist ferner ein Datenkabel mit entsprechendem Stecker zum Anschluss an eine Recheneinheit auf.

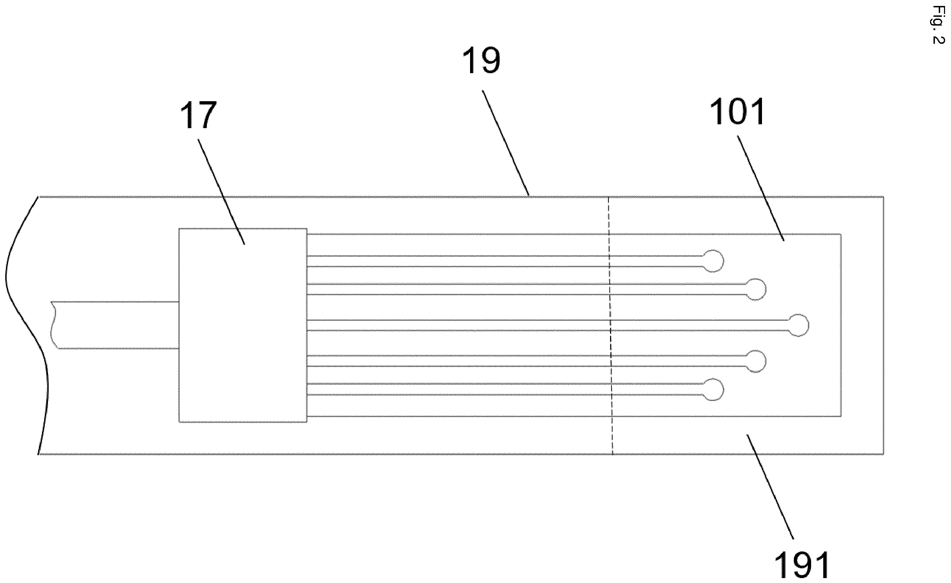

In Figur 2 ist dargestellt, wie die ionenselektive Sensoreinheit 101 mit angebrachter Anschlusseinrichtung 17 in dem Gehäuse 19 untergebracht ist, wobei hier das Gehäuse 19 offen dargestellt ist. Das Gehäuse 19 kann einstückig ausgeführt sein, in einer bevorzugten Ausführungsform, die auch hier dargestellt ist, ist das Gehäuse 19 zweiteilig, was die Montage etwas erleichtert.

Mit einer gestrichelten Linie ist das Stützteil 191 des Gehäuses 19 dargestellt, auf welchem der vordere Teil der ionenselektive Sensoreinheit 101 mit der Rückseite aufliegt, die Messpunkte liegen zur Vorderseite hin frei. Dieser Teil des Gehäuses bzw. der ionenselektiven Sensoreinheit 101 wird von der zu messenden Flüssigkeit umspült bzw. überspült, während der hier links der gestrichelten Linie dargestellte Teil fest und dicht verschlossen wird.

In Figur 3 wird schematisch der Multi-Ionen-Sensor 1 der bevorzugten Ausführungsform der Erfindung in geschlossenem Zustand dargestellt. Hier ist gestrichelt die ionenselektive Sensoreinheit 101 gezeigt, die auf dem Stützteil 191 des Gehäuses 19 aufliegt. Gegenüber ist der Messkopf 103 durch eine rückspringende Teilfläche 193 ausgebildet, so dass sich dazwischen der Messspalt S ausbilden kann. Die Breite B und die Höhe H sind eingezeichnet

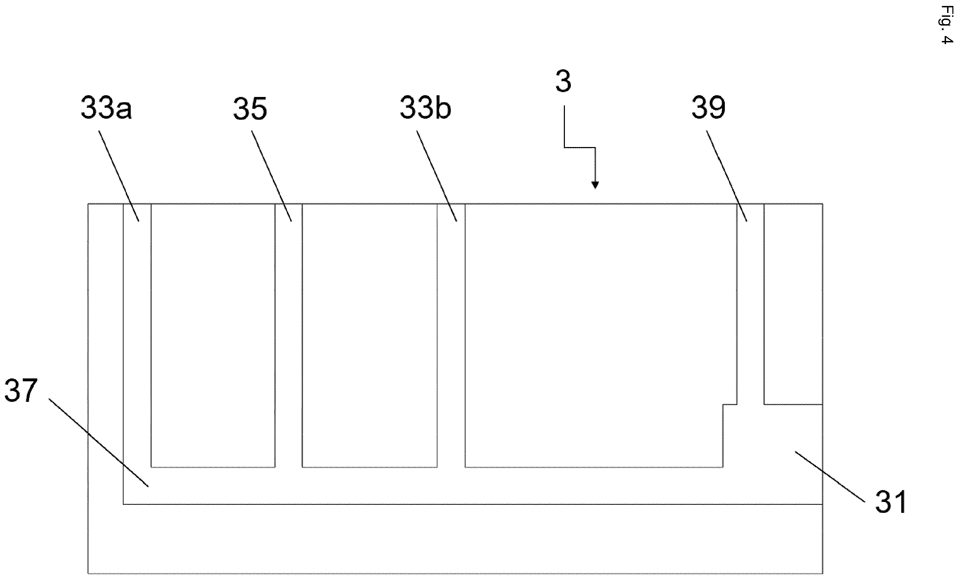

In Figur 4 wird die erfindungsgemäße Durchfluss-Messzelle 3 schematisch gemäß einer Ausführungsform der Erfindung im Schnitt dargestellt. Grundsätzlich ist eine Kalibrier-Zuleitung 33, durch die zwei Kalibierflüssigkeiten eingeführt werden können, ausreichend. Es hat sich aber in der Praxis als vorteilhaft herausgestellt, wenn für jede der beiden Kalibierflüssigkeiten eine eigene Kalibrier-Zuleitung 33a, 33b vorgesehen wird. In der vorliegenden Ausführungsform befindet sich die Proben-Zuleitung 35 zwischen den beiden Kalibrier-Zuleitungen 33a, 33b, das muss aber nicht zwingend der Fall sein. Zudem könnte abhängig von den Anforderungen auch eine weitere Proben-Zuleitung 35 vorgesehen werden. Die Zuleitungen 33a, 33b, 35 werden in der Sammelleitung 37 zusammengeführt und zur Aufnahmeöffnung 31 hingeführt.

Diese Aufnahmeöffnung 31 ist dazu ausgelegt, den Messkopf 103 des Multi-Ionen-Sensors 1 formschlüssig aufzunehmen, so dass keine Flüssigkeit an dieser Stelle aus der Durchfluss-Messzelle 3 austritt. Der Messspalt S bildet zusammen mit der Rückwand der Aufnahmeöffnung 31 einen Durchfluss-Messraum, in dem eine laminare Strömung ausgebildet werden kann. Dies wird dadurch unterstützt, dass die Sammelleitung 37 unten in die Aufnahmeöffnung 31 führt, während ober halb derselben die Ableitung 39 angeordnet ist.

Die vorliegende Erfindung dient der Messung von Ionen in wässrigen Systemen, insbesondere, aber nicht darauf beschränkt, der Überwachung von Ionen-Konzentrationen in Aquarien, hier vor allem Meerwasseraquarien.

Das System zur Messung von Ionen in wässrigen Systemen und zum Einstellen der Ionengehalte in den wässrigen Systemen umfasst die erfindungsgemäße Durchfluss-Messzelle 3 mit darin installiertem erfindungsgemäßem Multi-Ionen-Sensor 1. Eine Pumpeneinheit ist eingangsseitig zur Entnahme der Probe mit dem wässrigen System verbunden, hier konkret beispielsweise mit einem Aquarium. Eingesetzt werden Dosierpumpen, die kleine Volumina sehr präzise fördern können.

In den Schläuchen vom Aquarium zum Mess-System in die Messzelle sind in der Regel mehrere Milliliter Probe, d.h. hier Aquarienwasser, vorhanden. Vor einer Messung sollte zum Erhalt eines aktuellen Wertes frisches Probenwasser zugeführt werden. Um das in den Schläuchen vorhandene Wasser nicht zu verschwenden, indem es als Abwasser ausgegeben wird, ist vorgesehen, dass zunächst eine vorbestimmte Menge Probenwasser durch die Schläuche gepumpt wird, wobei das vorher in den Schläuchen stehende Wasser dem Aquarium wieder zugeführt wird. Anschließend kann über ein Umschaltventil der Fluss des Probenwassers in das Mess-System, konkret in die Durchfluss-Messzelle 3 geleitet werden.

Da jede ionenselektive Elektrode mit der Zeit driftet, wird für eine zuverlässige Messung vor jeder Messung kalibriert. Hierzu wird eine erste Kalibierflüssigkeit durch die Durchfluss-Messzelle 3 geleitet, deren Konzentration unterhalb der erwarteten und zu messenden Konzentration des Probenwassers liegt. Als nächstes wird dann das Probenwasser durch die Durchfluss-Messzelle 3 geleitet und die entsprechenden Messwerte als Messwertebündel aufgenommen. Schließlich wird eine zweite Kalibierflüssigkeit durch die Durchfluss-Messzelle 3 geleitet, deren Konzentration oberhalb der erwarteten und zu messenden Konzentration des Probenwassers liegt.

Die Flüssigkeitsvolumina der beiden Kalibierflüssigkeiten und des gemessenen Probenwassers werden verworfen und dem Abwasser zugeführt. Nach Abschluss der Messung wird der Durchfluss angehalten, ohne dass die Durchfluss-Messzelle 3 entleert werden muss und ohne dass der Multi-Ionen-Sensor 1 trocken fällt. Hierin liegt ein weiterer großer Vorteil der vorliegenden Erfindung.

### Bezugszeichen

- 1: Multi-Ionen-Sensor
- 11: Träger
- 101: ionenselektive Sensoreinheit
- 103: Messkopf
- 13: Referenzelektrode
- 15: Arbeitselektroden
- 17: Anschlusseinrichtung
- 19: Sensorgehäuse
- 191: Stützteil
- 193: rückspringende Teilfläche
- 3: Durchfluss-Messzelle
- 31: Aufnahmeöffnung
- 33: Kalibrier-Zuleitung
- 35: Proben-Zuleitung
- 37: Sammelleitung
- 39: Ableitung
- B: Breite
- H: Höhe
- S: Messspalt

## Patentansprüche

1. Multi-Ionen-Sensor (1) zur Messung von Ionen in wässrigen Systemen, umfassend
- einen flexiblen oder semi-flexiblen Träger (11),
- eine auf den Träger (11) aufgebrachte Referenzelektrode (13),
- mindestens drei auf den Träger (11) aufgebrachte Arbeitselektroden (15a, 15b, 15c),
wobei der Träger (11), die Referenzelektrode (13) und die Arbeitselektroden (15a, 15b, 15c) eine ionenselektive Sensoreinheit (101) bilden,
- eine Anschlusseinrichtung (17) für die ionenselektive Sensoreinheit (101) und
- ein Sensorgehäuse (19), welches eine Baugruppe aus ionenselektiver Sensoreinheit (101) und Anschlusseinrichtung (17) aufnimmt,
wobei die Referenzelektrode (13) Abmessungen von max. 4 mm x 4 mm x 4 mm aufweist.

2. Multi-Ionen-Sensor (1) nach Anspruch 1, wobei das Material der Referenzelektrode (13) ausgewählt ist aus polymerbeschichteten Fasern und Ag/AgCl.

3. Multi-Ionen-Sensor (1) nach Anspruch 1 oder 2, wobei das Sensorgehäuse (19) die Baugruppe aus ionenselektiver Sensoreinheit (101) und Anschlusseinrichtung (17) so aufnimmt, dass die Rückseite des Trägers (11) auf einem Stützteil (191) des Sensorgehäuses (19) aufliegt, und wobei das Sensorgehäuse (19) die Baugruppe aus ionenselektiver Sensoreinheit (101) und Anschlusseinrichtung (17) soweit umschließt, dass die Enden der Referenzelektrode (13) und der Arbeitselektroden (15a, 15b, 15c) gegenüber der Außenseite freiliegen und damit den Messkopf (103) des Multi-Ionen-Sensors (1) bilden.

4. Multi-Ionen-Sensor (1) nach Anspruch 3, wobei das Sensorgehäuse (19) eine rückspringende Teilfläche (193) aufweist, welche geometrisch mit dem Stützteil (191) mit darauf aufliegender ionenselektiver Sensoreinheit (101) korrespondiert, so dass zwischen der ionenselektiven Sensoreinheit (101) auf dem Stützteil (191) und der rückspringenden Teilfläche (193) ein Messspalt (S) gebildet wird.

5. Multi-Ionen-Sensor (1) nach Anspruch 4, wobei die Höhe (H) des Messspalts (S) und die Breite (B) des Messspalts (S) in einem geometrischen Verhältnis H : B von1 : 1 bis 4 : 1 stehen.

6. Durchfluss-Messzelle (3) für die Messung von Ionen in wässrigen Systemen, umfassend
- eine Aufnahmeöffnung (31) zur Montage eines Multi-Ionen-Sensors (1) nach einem der Ansprüche 1 bis 5,
wobei der Messkopf (103) des Multi-Ionen-Sensors (1) formschlüssig in der Aufnahmeöffnung (31) montierbar ist und zusammen mit der Rückwand der Aufnahmeöffnung (31) einen Durchfluss-Messraum bilden kann,
- zumindest eine Kalibrier-Zuleitung (33),
- zumindest eine Proben-Zuleitung (35),
- eine Sammelleitung (37), welche die zumindest eine Kalibrier-Zuleitung (33) und die zumindest eine Proben-Zuleitung (35) aufnimmt und sich zu der Aufnahmeöffnung (31) hin erstreckt,
- eine Ableitung (39), die sich oberhalb an die Aufnahmeöffnung (31) anschließt.

7. Durchfluss-Messzelle (3) nach Anspruch 6, wobei die Durchmesser der Kalibrier-Zuleitung (33) und/oder der Proben-Zuleitung (35) und/oder der Sammelleitung (37) 2 mm bis 4 mm betragen.

8. Durchfluss-Messzelle (3) nach Anspruch 6 oder 7, wobei das kumulierte Volumen der Kalibrier-Zuleitung (33), der Proben-Zuleitung (35), der Sammelleitung (37) und des Durchfluss-Messraums 2 ml bis 5 ml beträgt.

9. Verfahren zur Messung von Ionen in wässrigen Systemen, umfassend die Schritte
a) Bereitstellen einer Durchfluss-Messzelle (3) nach einem der Ansprüche 6 bis 8 mit einem montierten Multi-Ionen-Sensor (1) nach einem der Ansprüche 1 bis 5,
b) Einleiten einer ersten wässrigen Kalibrierlösung in die Durchfluss-Messzelle (3) mit Umspülen der ionenselektiven Sensoreinheit (101) des Multi-Ionen-Sensors (1), dabei Ausführen einer ersten Kalibrierung und Aufnehmen eines ersten Referenz-Messwerts,
c) Einleiten einer wässrigen Probe in die Durchfluss-Messzelle (3) mit Umspülen der ionenselektiven Sensoreinheit (101) des Multi-Ionen-Sensors (1), dabei Aufnehmen eines Messwertebündels,
d) Einleiten einer zweiten wässrigen Kalibrierlösung in die Durchfluss-Messzelle (3) mit Umspülen der ionenselektiven Sensoreinheit (101) des Multi-Ionen-Sensors (1), dabei Ausführen einer zweiten Kalibrierung und Aufnehmen eines zweiten Referenz-Messwerts,
wobei die Schritte a), b) c) und d) quasi kontinuierlich ausgeführt werden, ohne die Durchfluss-Messzelle (3) zu entleeren,
e) Berechnen der Messwerte aus ersten und zweiten Referenz-Messwerten und Messwertebündel,
f) Verarbeitung der Messwerte in einer Recheneinheit.

10. Verfahren nach Anspruch 9, wobei das zur Messung benötigte Volumen der ersten wässrigen Kalibrierlösung, der wässrigen Probe und der zweiten wässrigen Kalibrierlösung 3 ml bis 10 ml beträgt.

11. Verfahren nach Anspruch 9 oder 10, wobei vor Schritt b) das in den Zuleitungen zur Durchfluss-Messzelle (3) vorhandene Flüssigkeitsvolumen vollständig durch frisches Flüssigkeitsvolumen des wässrigen Systems ausgetauscht wird, wobei das ausgetauschte vorhandene Flüssigkeitsvolumen dem wässrigen System wieder zugeführt wird.

12. System zur Messung von Ionen in wässrigen Systemen und zum Einstellen der Ionengehalte in den wässrigen Systemen, umfassend
- eine Durchfluss-Messzelle (3) nach einem der Ansprüche 6 bis 8,
- einen in der Durchfluss-Messzelle (3) montierten Multi-Ionen-Sensor (1) nach einem der Ansprüche 1 bis 5,
- eine Pumpeneinheit, welche eingangsseitig zur Entnahme der Probe mit dem wässrigen System und mit Vorratsbehältern für die erste und zweite wässrige Kalibrierlösung fluiddynamisch verbunden ist und welche ausgangsseitig mit der zumindest einen Kalibrier-Zuleitung (33) und der zumindest einen Proben-Zuleitung (35) fluiddynamisch verbunden ist,
- eine Steuer-/Regel-Einheit, welche eingangsseitig mit dem Multi-Ionen-Sensor (1) elektronisch verbunden ist,
wobei die Steuer-/Regel-Einheit eine Auswerte-Elektronik zur Verarbeitung der ersten und zweiten Referenz-Messwerte und Messwertebündel aufweist.

13. System nach Anspruch 12, wobei die Steuer-/Regel-Einheit dazu ausgelegt ist, die Ionen-Konzentration in dem wässrigen System in Abhängigkeit der Regeldifferenz zu regulieren.
